# FASCICULE DE BREVET EUROPEEN

(11) **EP 2 588 591 B1**
(45) Date de publication et mention de la délivrance du brevet: **18.03.2015**
(21) Numéro de dépôt: 11743279.9
(22) Date de dépôt: 01.07.2011
(51) Int. Cl.: C12M 1/34

(54) **DISPOSITIF CAPTEUR D'UN PARAMETRE LIE A UN PHENOMENE ELECTRIQUE D'UN CONTENU BIOPHARMACEUTIQUE ET CONTENEUR BIOPHARMACEUTIQUE COMPORTANT UN TEL DISPOSITIF CAPTEUR.**
VORRICHTUNG ZUR MESSUNG EINES PARAMETERS IM ZUSAMMENHANG EINES ELEKTRISCHEN PHÄNOMENS EINES BIOPHARMAZEUTISCHEN INHALTS UND BIOPHARMAZEUTISCHER BEHÄLTER MIT EINER DERARTIGEN MESSVORRICHTUNG
DEVICE FOR SENSING A PARAMETER RELATED TO AN ELECTRICAL PHENOMENON OF A BIOPHARMACEUTICAL CONTENT AND BIOPHARMACEUTICAL CONTAINER COMPRISING SUCH A SENSING DEVICE

(30) Priorité: 02.07.2010 FR 1055403
(43) Date de publication de la demande: 08.05.2013
(73) Titulaire: SARTORIUS STEDIM FMT SAS, 13400 Aubagne (FR)
(72) Inventeur: CHAUSSIN, Sébastien, F-13400 Aubagne (FR); HEULE, Martin, NL-3452 AG Vleuten (NL)
(74) Mandataire: Derambure, Christian
(86) Numéro de dépôt international: PCT/FR2011/051541
(87) Numéro de publication internationale: WO 2012/001322

(56) Documents cités:
- EP-A2- 0 810 281
- WO-A1-2005/068059
- WO-A1-2008/153401
- WO-A2-2010/010313

## Description

L'invention est relative à la captation d'un paramètre lié à un phénomène électrique d'un contenu biopharmaceutique se trouvant dans l'espace intérieur d'un conteneur biopharmaceutique, apte à être traduit en un signal électrique.

Elle a plus spécialement pour objet, d'une part, un dispositif capteur d'un paramètre lié à un phénomène électrique et apte à être traduit en un signal électrique d'un contenu biopharmaceutique se trouvant dans l'espace intérieur d'un conteneur biopharmaceutique et, d'autre part, un conteneur biopharmaceutique comportant un tel dispositif capteur.

On entend par « paramètre lié à un phénomène électrique », un paramètre qui soit est *per se* de nature électrique, comme la conductivité, l'impédance ou la capacitance, soit est obtenu par un processus fonction d'une variable de nature électrique, comme la température (grâce à un thermocouple ou une sonde à résistance).

Le document WO 2008/153401 décrit un bioréacteur souple dont la paroi souple en partie inférieure est interrompue par un élément rigide relié de façon étanche à la paroi souple, l'élément rigide comprenant des moyens de mesure de l'activité cellulaire dans le bioréacteur en fonctionnement de manière non invasive. Ces moyens de mesure visent à mesurer l'oxygène, le pH ou la conductivité. Le bioréacteur est destiné à être porté et fixé à une plateforme basculante. Dans une première réalisation, ces moyens de mesure sont disposés selon une avancée pénétrant directement dans le contenu du bioréacteur et faisant face directement avec lui. Dans une seconde réalisation, il est prévu un réceptacle de mesure dans lequel sont placés les moyens de mesure. Dans cette réalisation, les moyens de mesure sont entourés d'une paroi fermée latéralement constituant le réceptacle, lequel est totalement ouvert dans sa partie extrême intérieure. Ainsi, ce réceptacle de mesure contient toujours une certaine quantité de liquide, même lorsque le bioréacteur bascule avec la plateforme basculante. Dans un cas comme dans l'autre, il n'est pas prévu la présence d'un organe de mélange tel qu'une hélice à l'intérieur même du bioréacteur et par conséquent, il n'y a pas d'impact sur la mesure du fait d'un tel organe de mélange ici inexistant qu'il conviendrait de pallier.

Le document EP 0 810 281 décrit un appareil de détection de contrôle du développement d'une culture dans un bioréacteur qui comprend en partie haute du bioréacteur une chambre dans laquelle sont placés une pluralité de capteurs, cette chambre étant ouverte frontalement. Il n'est pas davantage prévu la présence d'un organe de mélange tel qu'une hélice à l'intérieur même du bioréacteur.

Le document WO 2010/010313 décrit un bioréacteur analogue, dont la partie de captation d'impédance de type capacitif est en contact direct avec l'intérieur du bioréacteur.

Le document WO 2005/068059 décrit une poche souple de mélange destinée à recevoir des fluides biopharmaceutiques dans laquelle se trouvent des moyens de mélange et des moyens d'aération du contenu de la poche et des moyens capteur d'un paramètre tel que le pH, le taux d'oxygène, la température, etc. également disposés selon une avancée pénétrant directement dans le contenu de la poche.

Le document US 2007/0157748 décrit un agencement de fixation d'un capteur sur une pièce support fixée à la paroi souple d'une poche de mélange.

Le document WO 2009/071829 décrit un dispositif de raccordement à un récipient d'un accessoire ayant une partie active.

On a constaté que lorsqu'un dispositif capteur d'un paramètre lié à un phénomène électrique est associé à un conteneur biopharmaceutique dans lequel le contenu biopharmaceutique est sujet à une agitation - et plus spécialement à une assez puissante agitation - par suite de la mise en oeuvre d'un organe de mélange situé à l'intérieur même du conteneur, le fonctionnement du dispositif capteur est perturbé par cette agitation de sorte que la valeur du paramètre mesurée par le dispositif capteur peut ne pas être en permanence la valeur réelle exacte du paramètre.

L'invention a donc pour but d'apporter une solution à ce problème.

À cet effet, et selon un premier aspect, l'invention a pour objet un dispositif capteur d'un paramètre lié à un phénomène électrique et apte à être traduit en un signal électrique, tel que d'un contenu biopharmaceutique se trouvant dans l'espace d'un conteneur biopharmaceutique limité par une paroi ayant une ouverture, le contenu pouvant être sujet à une agitation, le dispositif capteur ayant un axe, un côté proximal vers le conteneur et un côté distal à l'opposé du conteneur.

Ce dispositif capteur comprend :
- des moyens de support et de fixation de type paroi, rigides, aptes à supporter les organes constitutifs du dispositif capteur et à être fixés rigidement et de façon étanche à la paroi à la périphérie de l'ouverture comprenant une paroi de support et de fixation et un ensemble de parois de chambre, délimitant une chambre pour le contenu, ayant une partie fermée incluant une partie ouverte apte à assurer le passage du contenu entre la chambre et l'espace intérieur du conteneur extérieur à elle, ledit ensemble de parois comprenant une paroi d'extrémité extérieure de chambre, fermée, disposée transversalement, apte à venir au droit de l'ouverture et au moins une paroi périphérique de chambre, s'étendant à partir de sa partie extrême distale en direction axiale vers le côté proximal à partir de la périphérie de la paroi d'extrémité extérieure, et
- des moyens de captation du paramètre, rigidement fixés aux moyens de support et de fixation, comportant une face de captation située dans la chambre, apte à être en contact électrique avec le contenu et des moyens de connexion électrique, reliés électriquement aux moyens de captation, rigidement fixés aux moyens de support et de fixation, aptes à être accessibles depuis l'extérieur du conteneur lorsque le dispositif capteur est fixé à lui et à être connectés électriquement à des moyens de raccordement électrique situés à l'extérieur du conteneur.

Ce dispositif capteur est tel que le dit ensemble de parois comprend, formant un tout rigide, une paroi d'extrémité intérieure de chambre, pleine c'est-à-dire fermée, disposée au moins pour partie généralement transversalement au droit de la paroi d'extrémité extérieure à partir de la partie extrême axiale proximale de la paroi périphérique, la partie ouverte étant ménagée dans la paroi périphérique. Ainsi, la chambre est une chambre de tranquillité apte à assurer un confinement du contenu qui s'y trouve, isolée de l'espace intérieur et préservée de l'agitation qui y règne par la paroi d'extrémité intérieure de chambre pleine c'est-à-dire fermée, de sorte à minimiser l'impact de cette agitation sur la mesure du paramètre du contenu par la face de captation.

Ainsi, il est prévu une paroi d'extrémité intérieure de chambre et celle-ci est pleine c'est-à-dire fermée, alors que la partie ouverte est ménagée dans la paroi périphérique et non dans la paroi d'extrémité intérieure de chambre.

Selon une réalisation, la paroi de support et de fixation est au moins pour partie de forme générale plane, limitée par un bord extérieur périphérique, dont l'une de ses faces comporte une zone périphérique de fixation rigide et étanche à la paroi du conteneur à la périphérie de son ouverture.

Selon une réalisation, la paroi de support et de fixation et la paroi d'extrémité extérieure sont deux parties fonctionnellement distinctes d'une même paroi.

Selon une réalisation, la paroi d'extrémité extérieure et la paroi de support et de fixation sont décalées en direction générale axiale et reliées l'une à l'autre par une paroi périphérique de liaison, s'étendant au moins pour partie généralement en direction axiale, s'étendant par rapport à la paroi de support et de fixation vers le côté distal ou vers le côté proximal.

Selon une réalisation, la face de captation est toute entière située dans la chambre de tranquillité sur au moins une face de la paroi d'extrémité extérieure et/ou d'une paroi de l'ensemble de parois, et est tournée vers l'intérieur de la chambre de tranquillité. Ainsi, la face de captation peut être située sur la paroi périphérique et/ou sur la paroi d'extrémité intérieure.

Selon une réalisation, la face de captation est située au droit d'une partie fermée de l'ensemble de parois et/ou de la paroi d'extrémité extérieure.

Selon une réalisation, la face de captation est sensiblement plane et coplanaire de la face interne sensiblement plane de la paroi sur laquelle elle est située.

Selon une réalisation, la face de captation est proche d'une partie fermée d'une paroi en regard de l'ensemble de parois ou de la paroi d'extrémité extérieure de chambre et/ou est écartée d'une partie ouverte.

Selon une réalisation, le dispositif comporte une pluralité de moyens de captation d'un ou de plusieurs paramètres. Par exemple, ces faces de captation sont situées sur la face tournée vers l'intérieur de la chambre de tranquillité d'une même paroi ou de parois différentes.

Selon une réalisation, les moyens de connexion électrique sont portés par la paroi de support et de fixation et/ou la paroi d'extrémité extérieure et/ou la paroi périphérique de liaison.

Selon une réalisation, les moyens de connexion électrique sont logés dans une cavité de protection. Par exemple, la cavité de protection est délimitée par la paroi d'extrémité extérieure et soit la paroi périphérique de liaison soit une paroi dédiée de cavité.

Selon une réalisation, le dispositif capteur comporte une pluralité de moyens de connexion électrique.

Selon une réalisation, les moyens de captation sont des moyens de captation de la conductivité, de l'impédance, de la capacitance, de la température du contenu biopharmaceutique.

Selon une réalisation, la paroi de support et de fixation présente une certaine tenue rigide.

Selon une réalisation, les moyens de support et de fixation incluent au moins un moyen support ou guide apte à supporter ou guider au moins un tourillon d'un moyen de mélange. Par exemple, le au moins un moyen support ou guide est situé sur ou porté par la paroi de support et de fixation et/ou l'ensemble de parois de chambre. Par exemple, le au moins un moyen support ou guide forme avec la paroi de support et de fixation et/ou l'ensemble de parois de chambre un palier borgne d'extrémité du au moins un tourillon d'un moyen de mélange.

Selon une réalisation, le dispositif capteur est à usage unique.

Selon un deuxième aspect, l'invention a pour objet un conteneur biopharmaceutique du type comportant une ou des entrées et sorties, au moins un moyen de mélange, le cas échéant au moins un moyen d'aération, un espace intérieur limité par une paroi ayant au moins une ouverture de passage et de montage pour au moins un dispositif capteur, le contenu biopharmaceutique du conteneur pouvant être sujet à une agitation, le au moins un dispositif capteur étant fixé rigidement et de façon étanche au conteneur autour de la au moins une ouverture de passage et apte à être en communication électrique d'une part avec le contenu et d'autre part avec des moyens de raccordement électrique situés à l'extérieur du conteneur, dans lequel le au moins un dispositif capteur est un dispositif capteur tel qu'il a été précédemment décrit.

Le au moins un moyen de mélange, le cas échéant au moins un moyen d'aération, comporte au moins un organe situé dans l'espace intérieur.

Selon une réalisation, un dispositif capteur est peu éloigné du au moins un moyen de mélange et le cas échéant du au moins un moyen d'aération.

Selon une réalisation, un dispositif capteur est situé en partie basse du conteneur biopharmaceutique.

Selon une réalisation, la paroi du conteneur biopharmaceutique présente une certaine souplesse.

Selon une réalisation, le conteneur est à usage unique.

Selon les réalisations, le conteneur biopharmaceutique constitue une poche de stockage et/ou remplissage et/ou manipulation et/ou transport et/ou mélange d'un contenu biopharmaceutique ou un bioréacteur d'un contenu biopharmaceutique.

On décrit maintenant plusieurs modes de réalisation de l'invention à l'aide des dessins, dans lesquels :

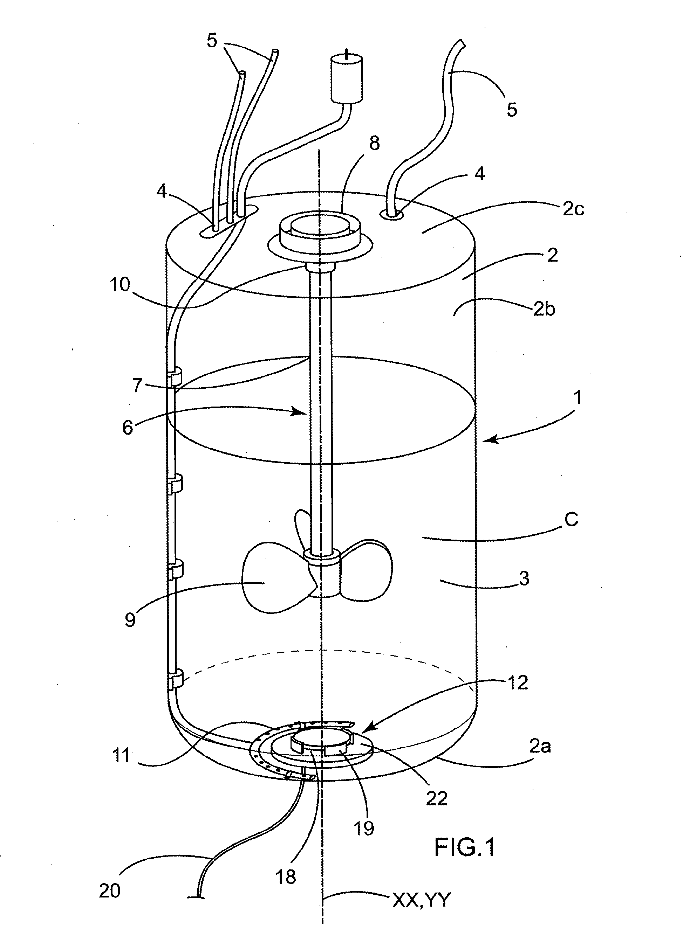

- La figure 1 est une vue schématique en perspective d'une réalisation possible d'un conteneur biopharmaceutique tel qu'un bioréacteur, dont le dispositif rigide extérieur de contention n'est pas représenté.

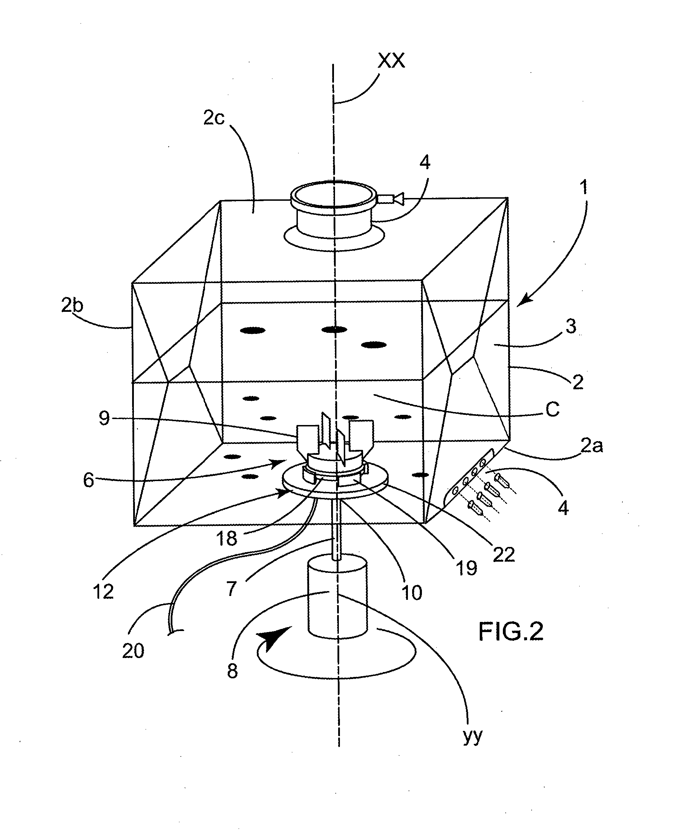

- La figure 2 est une vue schématique en perspective d'une réalisation possible d'un conteneur biopharmaceutique tel qu'une poche de stockage et/ou remplissage et/ou manipulation et/ou transport et/ou mélange d'un contenu biopharmaceutique, dont le dispositif rigide extérieur de contention n'est pas représenté.

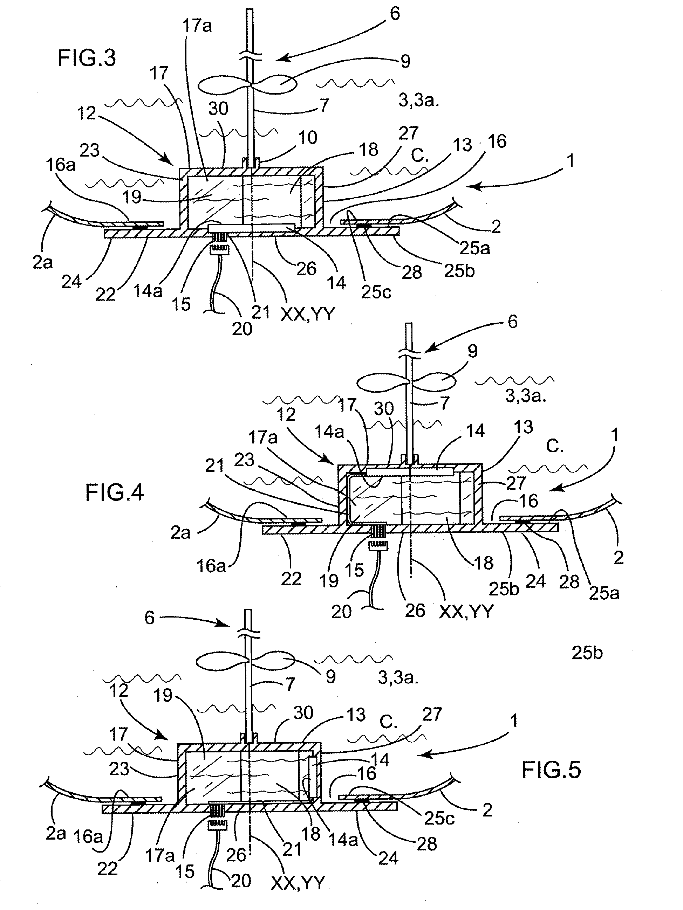

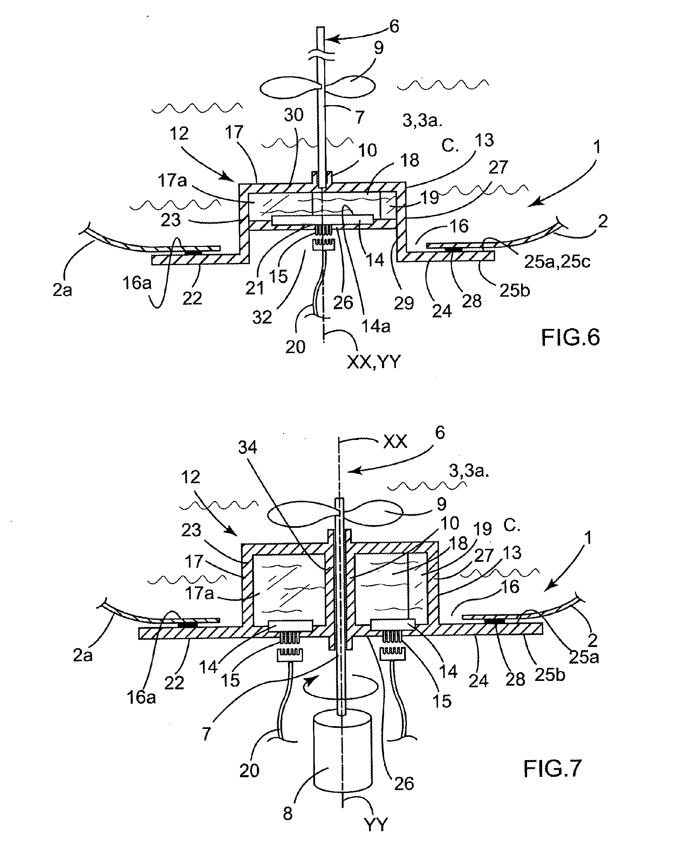

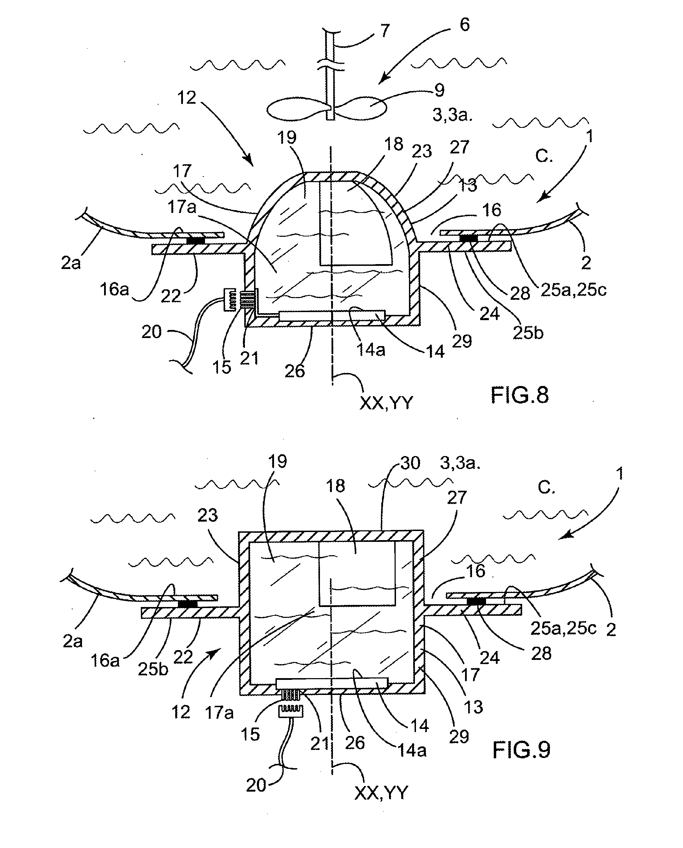

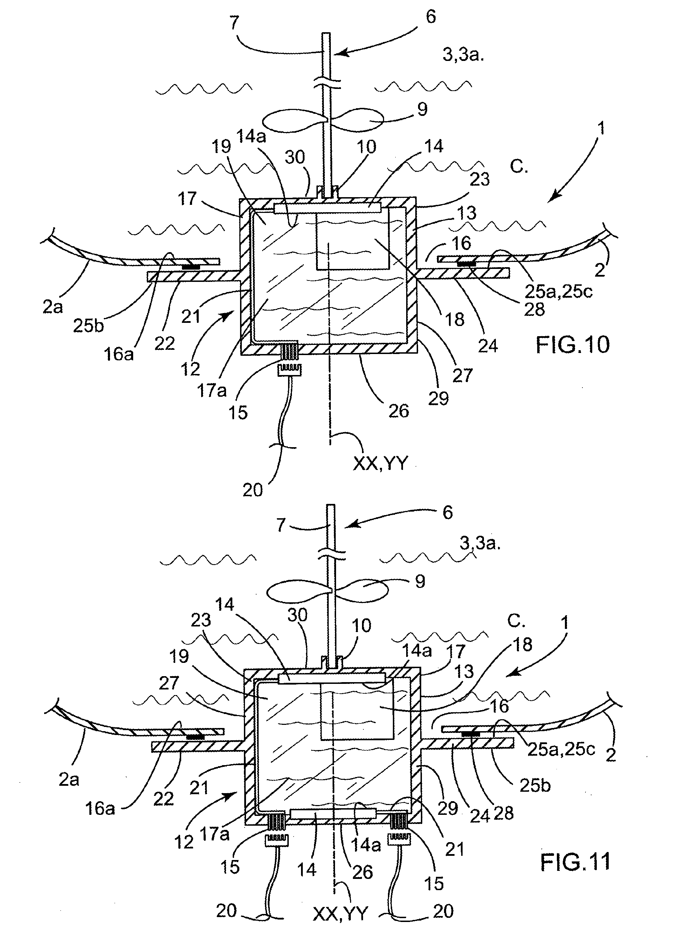

- Les figures 3 à 11 sont neuf vues schématiques, partielles, en coupe axiale de la partie inférieure d'un conteneur biopharmaceutique illustrant plus spécialement l'implantation d'un dispositif capteur d'un paramètre lié à un phénomène électrique et apte à être traduit en un signal électrique dont est pourvu un conteneur biopharmaceutique, ce dispositif capteur comportant une chambre de tranquillité, une face de captation située dans la chambre de tranquillité, des moyens de support et de fixation comprenant une paroi de support et de fixation et un ensemble de parois de chambre lui-même incluant une paroi d'extrémité axiale extérieure, une paroi périphérique et comme indiqué une paroi d'extrémité intérieure de chambre pleine c'est-à-dire fermée, le tout délimitant la chambre de tranquillité et ayant une ou des parties fermées incluant une ou des parties ouvertes situées dans la paroi périphérique, les figures correspondant à ces vues schématiques illustrant différentes réalisations possibles, indicatives et non limitatives susceptibles le cas échéant, sauf incompatibilité, à être combinées au moins pour partie entre elles, à savoir les réalisations dans lesquelles la paroi de support et de fixation et la paroi d'extrémité extérieure de chambre sont deux parties fonctionnellement distinctes d'une même paroi (figures 3 à 11), la paroi d'extrémité extérieure de chambre et la paroi de support et de fixation sont coplanaires (figures 3 à 5 et 7) ou décalées en direction axiale (figures 6 et 8 à 11), la paroi d'extrémité extérieure de chambre décalée est distale (figures 8 à 11) ou proximale (figure 6), la face de captation est située sur une paroi d'extrémité extérieure de chambre (figures 3, 6 à 9 et 11) ou sur une paroi périphérique de chambre (figure 5) ou sur une paroi d'extrémité intérieure de chambre (figures 4 et 10) ou sur plusieurs parois (figure 11), le dispositif capteur comporte un moyen de captation (figures 3 à 6, 8 à 10) ou plusieurs moyens de captation ou plusieurs moyens de connexion électrique (figures 7 et 11), les moyens de connexion électrique sont logés dans une cavité de protection (figure 6), les moyens de support et de fixation du dispositif capteur incluent un moyen support ou guide d'un tourillon d'un moyen de mélange (figures 3 à 7, 10 et 11), lequel forme un palier borgne d'extrémité (figures 3 à 6, 10 et 11) ou un palier traversant (figure 7), le dispositif capteur comporte une paroi annulaire formant protection pour le tourillon du moyen de mélange (figure 7).

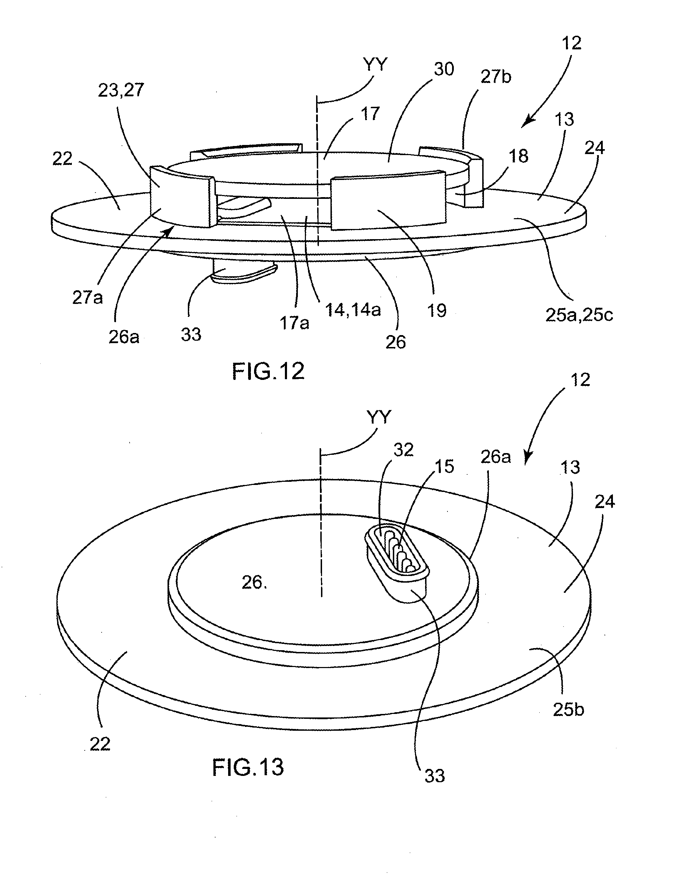

- Les figures 12 et 13 sont deux vues en perspective de côté d'une réalisation possible du dispositif capteur, respectivement vu du côté proximal et du côté distal, laquelle réalisation est proche structurellement de celle de la figure 3, si ce n'est que le dispositif capteur inclut un moyen support ou guide d'un tourillon d'un moyen de mélange dans le cas de la figure 3 mais non dans celui des figures 12 et 13, tandis que le dispositif capteur inclut une cavité de protection des moyens de connexion électrique dans le cas des figures 12 et 13, mais non dans celui de la figure 3.

Sur la figure 1 est représenté un conteneur biopharmaceutique 1 qui est ici un bioréacteur destiné à recevoir un contenu biopharmaceutique C comprenant une phase liquide et des cellules, des micro-organismes..., en vue d'une bioréaction qui peut être suivie et contrôlée par la mesure de la biomasse dans le bioréacteur 1. À cet effet, on vise à mesurer un paramètre représentatif de la biomasse tel que par exemple l'impédance.

Sur la figure 2 est représenté un conteneur biopharmaceutique 1 qui est ici une poche de stockage et/ou remplissage et/ou manipulation et/ou transport et/ou mélange destinée à recevoir un contenu biopharmaceutique C, comprenant au moins une phase liquide et, le cas échéant, un ou plusieurs composants pouvant être en phase solide, tel que de la poudre, en vue de son mélange. À cet effet, on vise à mesurer un paramètre représentatif du mélange tel que par exemple la conductivité.

En outre et le cas échéant, on vise à mesurer la température du contenu C.

Le conteneur 1 présente un axe principal XX qui, dans la position normale d'utilisation du conteneur 1 est situé de façon médiane et disposé verticalement.

Le conteneur 1, ayant une certaine flexibilité, est formé et limité par une paroi 2 présentant une certaine souplesse, en un ou plusieurs tronçons solidarisés les uns avec les autres par exemple par des soudures, ayant une partie inférieure 2a, une partie latérale 2b et une partie supérieure 2c, délimitant un espace intérieur 3, apte à recevoir une certaine quantité du contenu C.

Selon une réalisation, le conteneur 1 est à usage unique et la paroi 2 est un film en matière plastique.

Le conteneur 1 peut avoir une capacité plus ou moins grande, allant jusqu'à 5.000 litres, en fonction des besoins et des applications.

En raison de la nature flexible du conteneur 1, celui-ci, empli de son contenu C, est placé dans et maintenu par un récipient extérieur rigide ou éventuellement semi-rigide, non représenté, dont il épouse les faces internes, tandis que la géométrie, la forme, les dimensions du conteneur 1 sont adaptées à celles du récipient extérieur rigide.

Sauf si le contexte l'impose autrement, les mots « vertical », « horizontal », « supérieur », « inférieur », se réfèrent à la situation dans laquelle le conteneur 1 est dans une position apte au fonctionnement. Il est entendu toutefois que le conteneur 1 peut occuper d'autres positions ou avoir d'autres états, par exemple parce qu'il n'est pas en fonctionnement. Le mot « vertical » n'est pas limité à son sens littéral étroit mais signifie le plus souvent dans ce qui va du plus haut au plus bas ou inversement.

Le mot « axial » se réfère le plus souvent à ce qui s'étend dans la direction générale de l'axe concerné (axe XX ou axe YY). Le mot « transversal » se réfère le plus souvent à ce qui s'étend dans une direction générale orthogonale à l'axe concerné (axe XX ou axe YY).

De façon générale, les mots « intérieur » et « extérieur » se réfèrent respectivement à ce qui se trouve dans et hors du volume du moyen concerné. Sauf si le contexte l'impose autrement, ils visent le conteneur 1.

Le conteneur 1 comporte un ou une pluralité de ports 4 traversant d'introduction et de vidange, respectivement, aptes à être obturés ou au contraire ouverts selon les nécessités. Aux ports 4 peuvent être associés, en communication fluidique et étanche, le cas échéant amovible, des conduits, poches, réservoirs 5.

Dans la réalisation plus spécialement décrite ici, il est associé au conteneur 1 un moyen 6 de mélange du contenu C situé au moins pour partie dans l'espace intérieur 3. Un tel moyen 6 de mélange comporte un tourillon ou arbre 7, axial, apte à être entraîné à rotation par un moyen 8 moteur, et portant un ou plusieurs organes de mélange 9, tels que des pales. C'est cet organe 9 ou ces organes 9, ainsi que l'arbre 7 qui sont placés dans l'espace intérieur 3.

Il est prévu un ou plusieurs moyens 10 support ou guide apte à supporter ou à guider le tourillon 7. Un tel moyen 10 support ou guide est lui-même porté directement ou indirectement, en tout ou en partie, par la paroi 2 du conteneur 1.

Dans la réalisation représentée sur la figure 1, le moyen 8 moteur est placé en position supérieure au-dessus du conteneur 1, le tourillon 7 s'étend à partir de la partie supérieure 2c du conteneur 1, où se trouve un moyen 10 support ou guide, en direction de la partie inférieure 2a, et les organes de mélange 9 sont situés en partie médiane du conteneur 1, écartés de la partie inférieure 2b.

Dans la réalisation représentée sur la figure 2, le moyen 8 moteur est placé en position inférieure au-dessous du conteneur 1, le tourillon 7 s'étend à partir de la partie inférieure 2a du conteneur 1, où se trouve un moyen 10 support ou guide, en direction de la partie supérieure 2c, et les organes de mélange 9 sont situés au voisinage de la partie inférieure 2b du conteneur 1.

Le tourillon 7, le moyen 8 moteur, les organes de mélange 9 et le moyen 10 support ou guide peuvent faire l'objet de différentes réalisations à la portée de l'homme du métier. Il en est de même de leur arrangement et de leur association au conteneur 1.

Le cas échéant et comme illustré par la figure 1, le conteneur 1 peut comporter également un moyen d'aération 11 apte à délivrer au contenu C une certaine quantité de gaz d'aération situé au moins pour partie dans l'espace intérieur 3 et en particulier au moins pour sa partie extrême aval de sortie vers la partie inférieure 2a. Un tel moyen d'aération 11 peut faire l'objet de différentes réalisations à la portée de l'homme du métier. Il en est de même de son arrangement et de son association au conteneur 1.

Le conteneur 1 comporte également un (figures 1 à 6, 8 à 10 12 et 13) ou plusieurs (figures 7 et 11) dispositifs capteurs 12 d'un paramètre lié à un phénomène électrique et apte à être traduit en un signal électrique du contenu biopharmaceutique C, ayant un axe YY.

Un paramètre lié à un phénomène électrique est un paramètre qui soit est *per* se de nature électrique, comme la conductivité, l'impédance ou la capacitance, soit est obtenu par un processus fonction d'une variable de nature électrique, comme la température (grâce à un thermocouple ou une sonde à résistance).

Un tel dispositif capteur 12 comprend des moyens 13 de support et de fixation, des moyens 14 de captation du paramètre comportant une face de captation 14a apte à être en contact électrique avec le contenu C lorsque le dispositif capteur 12 est porté et associé au conteneur 1, et des moyens 15 de connexion électrique.

Par la suite, la description du dispositif capteur 12 est faite en référence au cas où celui-ci est associé et en position sur un conteneur 1. Toutefois, il va de soi que l'invention vise à la fois le dispositif capteur 12 seul, indépendamment de son montage sur un conteneur 1, et l'ensemble formé par le conteneur 1 et le dispositif capteur 12.

Pour permettre le montage du ou de chaque dispositif capteur 12 sur et dans le conteneur 1, la paroi 2 comporte une ou des ouvertures 16 de passage et de montage, ayant une périphérie 16a, avec laquelle ou avec lesquelles coopère ou coopèrent le ou les dispositifs capteur 12. L'ouverture 16 est bien entendu adaptée en taille au dispositif capteur 12 avec lequel elle est destinée à être associée. Elle est en outre adaptée en position sur le conteneur 1, à savoir située en partie inférieure 2a du conteneur 1, où est également situé le dispositif capteur 12. Ce faisant, le dispositif capteur 12 peut se trouver peu éloigné, voire même proche, du moyen 6 de mélange et/ou du moyen 11 d'aération, sans que son fonctionnement en soit perturbé.

Le dispositif capteur 12 présente un axe YY qui, dans les réalisations représentées sur les figures est coaxial de l'axe XX, l'axe YY étant également la direction axiale de l'ouverture de passage 16. Toutefois, l'invention vise également les cas où les axes XX et YY ne sont pas coaxiaux, étant soit parallèles entre eux et écartées l'un de l'autre d'une distance plus ou moins grande soit inclinés l'un par rapport à l'autre d'un angle plus ou moins grand.

Sauf si le contexte l'impose autrement, le mot « proximal » visant le dispositif capteur 12 ou tel organe constitutif de celui-ci, se réfère à ce qui est ou est propre à être dirigé vers le conteneur biopharmaceutique 1 (notamment l'intérieur du conteneur 1) lorsque le dispositif capteur 12 est monté sur celui-ci. Inversement, le mot « distal » se réfère à ce qui est ou est propre à être dirigé à l'opposé du conteneur biopharmaceutique 1 (notamment l'extérieur du conteneur 1).

Dans la réalisation plus spécialement décrite ici, le contenu C est sujet à une certaine agitation, voire à une agitation assez puissante, du fait du fonctionnement du moyen 6 de mélange ou du moyen 11 d'aération et de la présence d'un organe de celui-ci dans l'espace intérieur 3 au voisinage le cas échéant du dispositif capteur 12. Une telle agitation pourrait compromettre l'exactitude et la reproductibilité de la captation du paramètre par la face de captation 14a en contact électrique avec le contenu C. Pour pallier ce problème, il est prévu que le dispositif capteur 12 comporte, en outre, une chambre de tranquillité 17, définissant un espace intérieur 17a, apte à assurer un certain confinement du contenu C, la face de captation 14a étant positionnée dans la chambre de tranquillité 17, à savoir dans son espace intérieur 17a.

On désigne par la référence 3a la partie de l'espace intérieur 3 du conteneur 1 qui est extérieure à la chambre de tranquillité 17.

Ainsi, la chambre de tranquillité est isolée de l'espace intérieur 3a et préservée de l'agitation qui y règne, par une paroi 30 d'extrémité intérieure de chambre, pleine, c'est-à-dire fermée, sur laquelle on reviendra, de sorte à minimiser l'impact de cette agitation sur la mesure du paramètre du contenu C par la face de captation 14a.

D'une part, l'espace intérieur 17a de la chambre de tranquillité 17 est en communication fluidique avec l'espace intérieur 3a du conteneur 1 via une ou des parties ouvertes 18 de la chambre de tranquillité 17, aptes à assurer le passage du contenu C entre l'espace intérieur 17a de la chambre de tranquillité 17 et l'espace intérieur 3a du conteneur 1, et inversement, ainsi qu'une certaine homogénéisation du contenu C entre les deux espaces 3a et 17a.

D'autre part, l'espace intérieur 17a de la chambre de tranquillité 17 est agencé de manière à être isolé de l'espace intérieur 3a du conteneur 1 et la chambre de tranquillité 17 est agencée en conséquence, compte tenu du conteur 1, plus spécialement comportant la paroi 30 d'extrémité intérieure de chambre, pleine, c'est-à-dire fermée.

On entend par « isolé », le fait qu'il y ait entre l'espace intérieur 3a du conteneur 1 et l'espace intérieur 17a de la chambre de tranquillité 17, une séparation physique - la paroi 30 d'extrémité intérieure de chambre, pleine, c'est-à-dire fermée -, apte à - et ayant pour fonction - que l'agitation qui règne dans l'espace intérieur 3a soit très nettement atténuée, voire presque supprimée, dans l'espace intérieur 17a. Cette séparation physique est réalisée au moyen de la paroi 30 d'extrémité intérieure de chambre, pleine, c'est-à-dire fermée, et des parties fermées 19 de la chambre de tranquillité 17, qui incluent les parties ouvertes 18 situées ailleurs que sur la paroi 30 d'extrémité intérieure de chambre. C'est ainsi que l'on minimise l'impact de l'agitation régnant dans l'espace intérieur 3a du conteneur 1 sur la mesure du paramètre du contenu C par la face de captation 14a dans l'espace intérieur 17a de la chambre de tranquillité 17, même si le dispositif capteur 12 est proche du moyen 6 de mélange ou du moyen 11 d'aération.

Le dispositif capteur 12 est fixé rigidement et de façon étanche au conteneur 1, plus précisément à sa paroi 2, autour de l'ouverture de passage 16, plus précisément sur sa périphérie 16a, par exemple par un moyen de solidarisation 28 tel que moyen de collage ou moyen de soudage, interposé selon un contour continu entre eux. Ainsi, il présente, une fois monté sur le conteneur 1, un côté proximal propre à être dirigé vers le conteneur 1 et un côté distal propre à être dirigé à l'opposé du conteneur 1.

D'autre part, le dispositif capteur 12 est en communication électrique d'une part avec le contenu C par et grâce à la face de captation 14a et d'autre part, via les moyens 15 de connexion électrique, avec des moyens 20 de raccordement électrique situés à l'extérieur du conteneur 1. Des moyens 21 de communication électrique, tels que des contacts, des circuits imprimés ou analogue, assurent la communication électrique entre la face de captation 14a et les moyens 15 de connexion électrique, lesquels sont situés à proximité immédiate l'un de l'autre ou plus ou moins distants.

Les moyens 13 de support et de fixation sont de type paroi. Ils sont rigides et aptes à supporter les organes constitutifs du dispositif capteur 12 et à être fixés rigidement et de façon étanche à la paroi 2 du conteneur 1 à la périphérie 16a de son ouverture de passage 16 par le moyen de solidarisation 28.

Les moyens 14 de captation du paramètre et les moyens 15 de connexion électrique sont rigidement fixés aux moyens 13 de support et de fixation, notamment plus ou moins incorporés dans eux. Par exemple, les moyens 13 de support et de fixation sont intégrés ou incorporés d'une manière ou d'une autre sur les moyens 14 de captation du paramètre et les moyens 15 de connexion électrique, ou ces moyens 14 et 15 sont insérés dans les moyens 13 de support et de fixation.

Alors que les moyens 14 de captation du paramètre sont situés dans l'espace intérieur 3, et plus précisément dans l'espace intérieur 17a, les moyens 15 de connexion électrique sont situés à l'extérieur du conteneur 1 et sont aptes à être accessibles depuis l'extérieur du conteneur 1 lorsque le dispositif capteur 12 est fixé à lui.

La chambre de tranquillité 17 est délimitée par les moyens 13 de support et de fixation configurés avec une ou des parties fermées 19 incluant une ou des parties ouvertes 18, comme précédemment indiqué.

Les moyens 13 de support et de fixation comprennent au moins, une paroi 22 dite paroi de support et de fixation et, formant un tout rigide avec elle, dans sa zone médiane, un ensemble 23 de parois de chambre. Typiquement, la paroi 22 de support et de fixation et l'ensemble 23 de parois de chambre forment une pièce moulée ou injectée en matière plastique, monobloc, présentant, s'agissant de la paroi 22 de support et de fixation et de l'ensemble 23 de parois de chambre, une certaine tenue rigide.

La paroi 22 de support et de fixation est fermée, c'est-à-dire sans ouverture.

Au moins pour partie, la paroi 22 de support et de fixation est de forme générale plane.

Elle est disposée transversalement, au moins pour partie, par rapport à la direction axiale YY du dispositif capteur 12 et de l'ouverture de passage 16 du conteneur 1.

La paroi 22 de support et de fixation est limitée par un bord extérieur périphérique 24 et comporte deux faces 25a et 25b, dont l'une, 25a, comporte une zone périphérique 25c de fixation rigide et étanche à la paroi 2 du conteneur 1 à la périphérie 16a de l'ouverture de passage 16, et où se trouve le moyen de solidarisation 28.

En ce qui concerne les parois de l'ensemble 23 de parois de chambre, elles sont adjacentes entre elles et délimitent la chambre de tranquillité 17. En outre, les parois de l'ensemble 23 de parois de chambre comportent les parties fermées 19 incluant les parties ouvertes 18 précédemment mentionnées.

Les moyens 13 de support et de fixation comprenant la paroi 22 de support et de fixation et l'ensemble 23 de parois de chambre peuvent faire l'objet de différentes réalisations.

Dans les réalisations représentées, l'ensemble 23 de parois de chambre comprend, formant un tout rigide, au moins, une paroi 26 dite paroi d'extrémité axiale extérieure de chambre (ou paroi d'extrémité extérieure de chambre) et une paroi 27 dite paroi périphérique de chambre. Il comprend également la paroi 30 d'extrémité intérieure de chambre, pleine, c'est-à-dire fermée, déjà mentionnée.

La paroi 26 d'extrémité extérieure de chambre est pleine, c'est-à-dire fermée, sans ouverture. Elle est disposée transversalement, au moins pour partie, par rapport à la direction axiale YY du dispositif capteur 12 et de l'ouverture de passage 16 du conteneur 1. Elle est apte à venir au droit de l'ouverture de passage 16 du conteneur 1 et à pour fonction, au moins, de contribuer à la fermer. La paroi 26 est qualifiée d'extérieure dans la mesure où l'une de ses faces est située à l'extérieur du conteneur 1, tandis que l'autre face, opposée, est située dans l'espace intérieur 17a de la chambre de tranquillité 17, la paroi 26 étant ainsi également constitutive de la paroi limitant le conteneur 1. La paroi 26 est qualifiée d'extrémité axiale extérieure de chambre dans la mesure où elle forme la partie extrême fermée de la chambre de tranquillité 17 qui est distale et située vers l'extérieur du conteneur 1.

La paroi 27 périphérique de chambre, s'étend à partir de la périphérie 26a (figure 12) de la paroi 26 d'extrémité extérieure de chambre 26. A partir de sa partie extrême axiale distale 27a (figure 12), attenante à la paroi 26 d'extrémité extérieure de chambre, la paroi 27 périphérique de chambre s'étend au moins pour partie généralement en direction axiale YY vers le côté proximal du conteneur 1, c'est-à-dire vers l'intérieur de ce dernier, et comporte une partie extrême axiale proximale 27b (figure 12).

On se réfère maintenant plus spécialement aux figures 3 et 13 qui représentent deux réalisations proches fonctionnellement. Dans ces réalisations, la paroi 22 de support et de fixation et la paroi 26 d'extrémité extérieure de chambre sont les deux parties sensiblement coplanaires structurellement distinctes d'une même paroi, à savoir sa périphérie annulaire pour la paroi 22 et sa partie centrale pour la paroi 26. On trouve une même disposition dans les réalisations représentées sur les figures 4, 5 et 7.

Dans d'autres réalisations (figures 6, 8 à 11), la paroi 26 d'extrémité extérieure de chambre et la paroi 22 de support et de fixation sont décalées en direction générale axiale (le long de l'axe YY) et elles sont reliées l'une à l'autre par une paroi 29 dite paroi périphérique de liaison, laquelle s'étend, au moins pour partie, en direction générale axiale.

Par exemple, dans la réalisation de la figure 6, la paroi 26 d'extrémité extérieure de chambre est proximale, tandis que, par rapport à la paroi 22 de support et de fixation, la paroi 29 périphérique de liaison s'étend vers le côté proximal.

Dans les réalisations des figures 8 à 11, au contraire, la paroi 26 d'extrémité extérieure de chambre est distale, tandis que, par rapport à la paroi 22 de support et de fixation, la paroi 29 périphérique de liaison s'étend vers le côté distal.

On se réfère maintenant plus spécialement aux figures 3 à 7 et 9 à 11, dans lesquelles l'ensemble 13 de parois de chambre comprend en outre, formant un tout rigide, la paroi 30 d'extrémité axiale intérieure de chambre (ou paroi d'extrémité intérieure de chambre) qui est ici disposée transversalement par rapport à la direction axiale YY du dispositif capteur 12 et de l'ouverture de passage 16 du conteneur 1. La paroi 30 d'extrémité axiale intérieure de chambre est située au moins pour partie au droit de la paroi 26 d'extrémité extérieure de chambre. La paroi 30 d'extrémité axiale intérieure de chambre est attenante à la partie extrême proximale 27a de la paroi 27 périphérique de chambre. Dans ces différentes réalisations, les parties ouvertes 18 sont ménagées dans la paroi 27 périphérique de chambre, mais non dans la paroi 30 d'extrémité intérieure de chambre qui est pleine, c'est-à-dire fermée.

On se réfère maintenant plus spécialement à la figure 8 dans laquelle l'ensemble 13 de parois de chambre comprend également une paroi 30 ayant un prolongement bombé de raccordement avec la paroi 27 périphérique de chambre, les parties ouvertes 18 étant ménagées latéralement, mais non dans la paroi 30 d'extrémité intérieure de chambre qui est pleine, c'est-à-dire fermée.

La chambre de tranquillité 17 s'étend par conséquent entre, d'un côté axial, la paroi 26 d'extrémité extérieure de chambre et de l'autre côté axial, la paroi 30 d'extrémité intérieure de chambre, toutes deux pleines c'est-à-dire fermées, et latéralement, la paroi 27 périphérique de chambre qui, soit assure cette seule fonction (figures 3 à 7) soit assure en tout ou partie celle de la paroi 29 périphérique de liaison (figures 8 à 11). Dans tous les cas, la paroi 27 périphérique de chambre comporte les parties 28 ouvertes.

Comme indiqué, la face de captation 14a du paramètre est positionnée dans la chambre de tranquillité 17, à savoir dans son espace intérieur 17a et, ainsi, elle est en contact électrique avec le contenu C se trouvant dans l'espace intérieur 17a de la chambre de tranquillité 17. Plus précisément, la face de captation 14a est toute entière située dans la chambre de tranquillité 17, sur au moins une face de la paroi 26 d'extrémité extérieure de chambre (figures 3 et 6 à 9) ou d'une paroi de l'ensemble 23 de parois de chambre (figures 4, 5 et 10) ou sur les deux (figures 11), laquelle face de captation 14a est tournée vers l'intérieur de la chambre de tranquillité 17 pour être située dans son espace intérieur 17a. Au regard de l'ensemble 23 de parois de chambre, la face de captation 14a est située sur la face tournée vers l'intérieur de la paroi 27 périphérique de chambre (figure 5) ou de la paroi 30 d'extrémité intérieure de chambre (figure 4 et 10) ou sur les deux.

Selon une réalisation (figures 3 à 11), la face de captation 14a est située au droit d'une partie fermée de l'ensemble 23 de parois de chambre et/ou de la paroi 26 d'extrémité extérieure de chambre.

Selon une caractéristique représentée sur les figures, correspondant à une réalisation de l'invention, la face de captation 14a est sensiblement plane et coplanaire de la face interne sensiblement plane de la paroi sur laquelle elle est située 26, 27, 30.

Selon une possibilité de réalisation, la face de captation 14a est proche d'une partie fermée 19 d'une paroi en regard de l'ensemble 23 de parois de chambre ou de la paroi 26 d'extrémité extérieure de chambre et/ou est écartée d'une partie ouverte 18. Cette disposition constructive a pour effet que l'agitation dans l'espace intérieur 17a de la chambre de tranquillité 17 est très atténuée, voire quasi inexistante, par rapport à celle existant dans l'espace intérieur 3a.

On se réfère aux figures 3 à 6 et 8 à 10 qui illustrent un dispositif capteur 12 qui comporte un seul moyen 14 de captation pour un paramètre. Dans le cas des figures 7 et 11, le dispositif capteur 12 comporte au contraire une pluralité de moyens 14 de captation. Cette pluralité de moyens 14 de captation est destinée à capter un seul paramètre (par exemple à des fins de sécurité) ou plusieurs paramètres. Une telle disposition est particulièrement utile lorsque la valeur d'un premier paramètre est liée à celle d'un second. Par exemple, on peut, grâce à cet agencement, capter la conductivité et la température à laquelle la conductivité a été captée.

Dans une telle réalisation et selon les agencements, les faces de captation 14a de la pluralité de faces de captation de la pluralité de moyens 14 de captation sont situées sur la face tournée vers l'intérieur de la chambre d'une même paroi - en l'espèce dans le cas de la figure 7, la paroi 26 d'extrémité extérieure de chambre - ou de parois différentes - en l'espèce dans le cas de la figure 11, la paroi 26 d'extrémité extérieure de chambre et la paroi 30 d'extrémité intérieure de chambre -. Plus généralement, la pluralité des faces de captation 14a sont situées sur les parois 26, 27, 29 et 30, selon les configurations.

Dans les réalisations des figures 3 à 7 et 9 à 11, les moyens 15 de connexion électrique sont portés par la paroi 26 d'extrémité extérieure de chambre. Dans la réalisation de la figure 8, les moyens de connexion électrique sont portés par la paroi 29 périphérique de liaison. Bien entendu, lorsqu'il est prévu plusieurs moyens 15 de connexion électrique, il est possible de combiner ces modes de réalisations les uns avec les autres.

Dans les réalisations des figures 6 et 13, les moyens 15 de connexion électrique sont logés dans une cavité de protection 32. Une telle cavité de protection est délimitée par la paroi 26 d'extrémité extérieure de chambre et soit la paroi 29 périphérique de liaison (figure 6) soit une paroi 33 dédiée de cavité (figure 13). Cette disposition constructive permet de protéger les moyens 15 de connexion électrique de toute détérioration qui pourrait résulter d'une force appliquée sur eux.

Selon d'autres réalisations (figures 3 à 5 et 7 à 11) il n'est pas prévu une telle cavité de protection des moyens 15 de connexion électrique.

Dans les réalisations des figures 3 à 6, 8 à 10 et 13, le dispositif capteur 12 comporte un seul ensemble de moyens 15 de connexion électrique. Dans les réalisations des figures 7 et 11, le dispositif capteur 12 comporte une pluralité de moyens 15 de connexion électrique. Cette dernière réalisation va le plus souvent de pair avec une même pluralité de moyens 14 de captation.

Dans la réalisation plus spécialement décrite ici, au conteneur 1 est associé le plus souvent un moyen 6 de mélange qui comporte un tourillon ou arbre 7 et un ou plusieurs moyens 10 support ou guide apte à supporter ou à guider le tourillon 7.

Selon un développement de l'invention, illustré par les figures 3 à 7, et 10 et 11, le dispositif capteur 12 et de tels moyens 10 support ou guide sont intégrés, les moyens 13 de support et de fixation incluant au moins un tel moyen 10 support ou guide. Plus particulièrement, un tel moyen 10 support ou guide est situé sur ou porté par la paroi 22 de support et de fixation et/ou l'ensemble 23 de parois de chambre. Selon les cas, et comme représenté sur les dessins, au dispositif capteur 12 est intégré un seul moyen 10 support ou guide. Dans d'autres cas (non représentés), à un dispositif capteur 12 donné sont intégrés plusieurs moyens 10 support ou guide pour plusieurs tourillon ou arbres 7, notamment disposés parallèlement entre eux.

On se réfère plus spécialement maintenant aux figures 3 à 6, et 10 et 11 qui illustrent une réalisation dans laquelle le moyen 10 support ou guide forme, avec la paroi 30 d'extrémité intérieure de chambre, un palier borgne d'extrémité du tourillon ou arbre 7. Le moyen 10 support ou guide, comme le tourillon ou arbre 7, est alors placé au dessus de la chambre de tranquillité 17, et en totalité à l'extérieur d'elle. Cette réalisation tire parti de ce que la paroi 30 d'extrémité intérieure de chambre est pleine, c'est-à-dire fermée.

On se réfère plus spécialement maintenant à la figure 7 qui illustre une réalisation dans laquelle le moyen 10 support ou guide forme avec la paroi 22 de support et de fixation et/ou avec l'ensemble 3 de parois de chambre, un palier traversant du tourillon ou arbre 7. Le moyen 10 support ou guide, comme le tourillon ou arbre 7, est alors situé pour partie à l'extérieur et pour partie à l'intérieur de la chambre de tranquillité 17. Pour éviter que le tourillon ou arbre 7 traversant la chambre de tranquillité 17 ne créé une agitation, alors que l'on cherche précisément à minimiser l'agitation, on peut prévoir, par exemple une paroi 34 annulaire, située au moins pour partie à l'intérieur de la chambre de tranquillité 17, laquelle paroi 34 forme une protection pour le tourillon ou arbre 7.

Grâce à l'agencement du moyen 10 support ou guide et du dispositif capteur 12, il, est possible, sans préjudice pour la captation du ou des paramètres, de placer le dispositif capteur 12 dans une localisation où il est peu éloigné du moyen 6 de mélange et le cas échéant du moyen 11 d'aération, voire où il est très proche de lui.

Pour réaliser des conteneurs biopharmaceutiques 1 pourvus de dispositifs capteur 12, on dispose d'une part de conteneur biopharmaceutique 1 nus et d'autre part de dispositifs capteur 12. Grâce à l'invention, ces dispositifs capteurs 12 ont pu être calibrés préalablement. Puis, on monte un dispositif capteur 12 sur un conteneur 1 en le positionnant dans l'ouverture 16 et, enfin, on solidarise de façon fixe et étanche la paroi 22 de support et de fixation à la paroi 2 dans la périphérie 16a.

En jouant sur la structure, la forme, la disposition et la taille de la chambre de tranquillité 17, il est possible de minimiser de façon très importante l'effet résultant de l'agitation provoquée par le moyen 6 de mélange ou le moyen 11 d'aération lorsqu'il existe.

## Revendications

1. Dispositif capteur (12) d'un paramètre lié à un phénomène électrique et apte à être traduit en un signal électrique, tel que d'un contenu biopharmaceutique (C) se trouvant dans l'espace (3) d'un conteneur biopharmaceutique (1) limité par une paroi (2) ayant une ouverture (16), le contenu (C) pouvant être sujet à une agitation, le dispositif capteur (12) d'axe (YY), de côté proximal vers le conteneur (1) et de côté distal à l'opposé du conteneur (1), comprenant :
- des moyens (13) de support et de fixation de type paroi, rigides, aptes à supporter les organes constitutifs du dispositif capteur (12) et à être fixés rigidement et de façon étanche à la paroi (2) à la périphérie (16a) de l'ouverture (16) comprenant une paroi (22) de support et de fixation et un ensemble (23) de parois de chambre, délimitant une chambre (17) pour le contenu (C), ayant une partie fermée (19) incluant une partie ouverte (18) apte à assurer le passage du contenu (C) entre la chambre (17) et l'espace intérieur (3a) du conteneur (1) extérieur à elle, l'ensemble (23) de parois comprenant une paroi (26) d'extrémité extérieure de chambre, fermée, disposée transversalement, apte à venir au droit de l'ouverture (16) et au moins une paroi (27) périphérique de chambre, s'étendant à partir de sa partie extrême distale (27a) en direction axiale vers le côté proximal à partir de la périphérie (26a) de la paroi (26) d'extrémité extérieure,
- des moyens (14) de captation du paramètre, rigidement fixés aux moyens (13) de support et de fixation, comportant une face de captation (14a) située dans la chambre (17), apte à être en contact électrique avec le contenu (C) et des moyens (15) de connexion électrique, reliés électriquement aux moyens (14) de captation, rigidement fixés aux moyens (13) de support et de fixation, aptes à être accessibles depuis l'extérieur du conteneur (1) lorsque le dispositif capteur (12) est fixé à lui et à être connectés électriquement à des moyens (20) de raccordement électrique situés à l'extérieur du conteneur (1),
**caractérisé par le fait que** l'ensemble (23) de parois comprend, formant un tout rigide, une paroi (30) d'extrémité intérieure de chambre, pleine, c'est-à-dire fermée, disposée au moins pour partie généralement transversalement au droit de la paroi (26) d'extrémité extérieure à partir de la partie extrême axiale proximale (27b) de la paroi (27) périphérique, la partie ouverte (18) étant ménagée dans la paroi (27) périphérique, la chambre (17) étant une chambre de tranquillité (17) apte à assurer un confinement du contenu (C) qui s'y trouve, isolée de l'espace intérieur (3a) et préservée de l'agitation qui y règne, par la paroi (30) d'extrémité intérieure de chambre, pleine, c'est-à-dire fermée, de sorte à minimiser l'impact de cette agitation sur la mesure du paramètre du contenu (C) par la face de captation (14a).

2. Dispositif capteur (12) selon la revendication 1, dans lequel la paroi (22) de support et de fixation est au moins pour partie de forme générale plane, limitée par un bord extérieur périphérique (24), dont l'une de ses faces (25a, 25b) comporte une zone périphérique (25c) de fixation rigide et étanche à la paroi (2) du conteneur (1) à la périphérie (16a) de son ouverture (16).

3. Dispositif capteur (12) selon l'une quelconque des revendications 1 et 2, dans lequel la paroi (22) de support et de fixation et la paroi (26) d'extrémité extérieure sont deux parties fonctionnellement distinctes d'une même paroi.

4. Dispositif capteur (12) selon l'une quelconque des revendications 1 à 3, dans lequel la paroi (26) d'extrémité extérieure et la paroi (22) de support et de fixation sont décalées en direction générale axiale et reliées l'une à l'autre par une paroi (29) périphérique de liaison, s'étendant au moins pour partie généralement en direction axiale, s'étendant par rapport à la paroi (22) de support et de fixation vers le côté distal ou vers le côté proximal.

5. Dispositif capteur (12) selon l'une quelconque des revendications 1 à 4, dans lequel la face de captation (14a) est toute entière située dans la chambre de tranquillité (17) sur au moins une face de la paroi (26) d'extrémité extérieure et/ou d'une paroi de l'ensemble (23) de parois, et est tournée vers l'intérieur de la chambre de tranquillité (17).

6. Dispositif capteur (12) selon la revendication 5, dans lequel la face de captation (14a) est située sur la paroi (27) périphérique et/ou sur la paroi (30) d'extrémité intérieure.

7. Dispositif capteur (12) selon l'une quelconque des revendications 5 et 6, dans lequel la face de captation (14a) est située au droit d'une partie fermée de l'ensemble (23) de parois et/ou de la paroi (26) d'extrémité extérieure.

8. Dispositif capteur (12) selon l'une quelconque des revendications 5 à 7, dans lequel la face de captation (14a) est sensiblement plane et coplanaire de la face interne sensiblement plane de la paroi sur laquelle elle est située.

9. Dispositif capteur (12) selon l'une quelconque des revendications 5 à 8, dans lequel la face de captation (14a) est proche d'une partie fermée (19) d'une paroi en regard de l'ensemble (23) de parois ou de la paroi (26) d'extrémité extérieure de chambre et/ou est écartée d'une partie ouverte (18).

10. Dispositif capteur (12) selon l'une quelconque des revendications 1 à 9, qui comporte une pluralité de moyens (14) de captation d'un ou de plusieurs paramètres.

11. Dispositif capteur (12) selon la revendication 10, dans lequel les faces de captation (14a) de la pluralité de faces de captation de la pluralité de moyens (14) de captation sont situées sur la face tournée vers l'intérieur de la chambre de tranquillité (17) d'une même paroi (26, 27, 29, 30) ou de parois différentes (26, 27,29,30).

12. Dispositif capteur (12) selon l'une quelconque des revendications 1 à 11, dans lequel les moyens (15) de connexion électrique sont portés par la paroi (22) de support et de fixation et/ou la paroi (26) d'extrémité extérieure et/ou la paroi (29) périphérique de liaison.

13. Dispositif capteur (12) selon l'une quelconque des revendications 1 à 12, dans lequel les moyens (15) de connexion électrique sont logés dans une cavité de protection (32).

14. Dispositif capteur (12) selon la revendication 13, dans lequel la cavité de protection (32) est délimitée par la paroi (26) d'extrémité extérieure et soit la paroi (29) périphérique de liaison soit une paroi (33) dédiée de cavité.

15. Dispositif capteur (12) selon l'une quelconque des revendications 1 à 14, qui comporte une pluralité de moyens (15) de connexion électrique.

16. Dispositif capteur (12) selon l'une quelconque des revendications 1 à 15, dans lequel les moyens (14) de captation sont des moyens (14) de captation de la conductivité, de l'impédance, de la capacitance, de la température du contenu biopharmaceutique (C).

17. Dispositif capteur (12) selon l'une quelconque des revendications 1 à 16, dans lequel la paroi (22) de support et de fixation présente une certaine tenue rigide.

18. Dispositif capteur (12) selon l'une quelconque des revendications 1 à 17, **caractérisé par le fait que** les moyens (13) de support et de fixation incluent au moins un moyen (10) support ou guide apte à supporter ou guider au moins un tourillon (7) d'un moyen (6) de mélange.

19. Dispositif capteur (12) selon la revendication 18, **caractérisé par le fait que** le au moins un moyen (10) support ou guide est situé sur ou porté par la paroi (22) de support et de fixation et/ou l'ensemble (23) de parois de chambre.

20. Dispositif capteur (12) selon la revendication 19, **caractérisé par le fait que** le au moins un moyen (10) support ou guide forme avec la paroi (22) de support et de fixation et/ou l'ensemble (23) de parois de chambre un palier borgne d'extrémité du au moins un tourillon (7) d'un moyen (6) de mélange.

21. Dispositif capteur (12) selon l'une quelconque des revendications 1 à 20, **caractérisé par le fait qu'**il est à usage unique.

22. Conteneur biopharmaceutique (1) du type comportant une ou des entrées et sorties (4), au moins un moyen de mélange (6), le cas échéant au moins un moyen d'aération, un espace intérieur (3) limité par une paroi (2) ayant au moins une ouverture de passage (16) et de montage pour au moins un dispositif capteur (12), le contenu biopharmaceutique (C) du conteneur (1) pouvant être sujet à une agitation, le au moins un dispositif capteur (12) étant fixé rigidement et de façon étanche au conteneur (1) autour de la au moins une ouverture de passage (16) et apte à être en communication électrique d'une part avec le contenu (C) et d'autre part avec des moyens (20) de raccordement électrique situés à l'extérieur du conteneur (1), dans lequel le au moins un dispositif capteur (12) est un dispositif capteur (12) selon l'une quelconque des revendications 1 à 21.

23. Conteneur biopharmaceutique (1) selon la revendication 22, dans lequel au moins un moyen de mélange (6), le cas échéant au moins un moyen d'aération, comporte au moins un organe (9) situé dans l'espace intérieur (3).

24. Conteneur biopharmaceutique (1) selon l'une quelconque des revendications 22 et 23, dans lequel un dispositif capteur (12) est peu éloigné du au moins un moyen (6) de mélange et le cas échéant du au moins un moyen (11) d'aération.

25. Conteneur biopharmaceutique (1) selon l'une quelconque des revendications 22 à 24, dans lequel un dispositif capteur (12) est situé en partie basse du conteneur biopharmaceutique (1).

26. Conteneur biopharmaceutique (1) selon l'une quelconque des revendications 22 à 25, dans lequel la paroi (2) du conteneur biopharmaceutique (1) présente une certaine souplesse.

27. Conteneur biopharmaceutique (1) selon l'une quelconque des revendications 22 à 26, **caractérisé par le fait qu'**il est à usage unique.

28. Conteneur biopharmaceutique (1) selon l'une quelconque des revendications 22 à 27 qui constitue une poche de stockage et/ou remplissage et/ou manipulation et/ou transport et/ou mélange d'un contenu biopharmaceutique (C) ou un bioréacteur d'un contenu biopharmaceutique (C).

## Patentansprüche

1. Vorrichtung (12) zur Messung eines Parameters, der mit einem elektrischen Phänomen zusammenhängt und geeignet ist, in ein elektrisches Signal umgewandelt zu werden, wie von einem biopharmazeutischen Inhalt (C), der sich in dem Raum (3) eines biopharmazeutischen Behälters (1) befindet, der durch eine Wand (2) abgegrenzt ist, die eine Öffnung (16) aufweist, wobei der Inhalt (C) einer Bewegung unterzogen werden kann, wobei die Messvorrichtung (12) mit der Achse (YY) auf der proximalen Seite in Richtung des Behälters (1) und auf der distalen, gegenüberliegenden Seite des Behälters (1) Folgendes aufweist:
- starre Stütz- und Befestigungsmittel (13) des Typs Wand, die geeignet sind, die Organe zu stützen, aus denen die Messvorrichtung (12) besteht, und starr und auf dichte Weise an der Wand (2) am Umfang (16a) der Öffnung (16) befestigt zu werden, die eine Stütz- und Befestigungswand (22) und eine Menge (23) von Kammerwänden aufweisen, die eine Kammer (17) für den Inhalt (C) abgrenzen, die einen geschlossenen Teil (19) aufweist, der einen offenen Teil (18) aufweist, der geeignet ist, den Durchgang des Inhalts (C) zwischen der Kammer (17) und dem Innenraum (3a) des außerhalb von ihr gelegenen Behälters (1) zu gewährleisten, wobei die Menge (23) von Wänden eine geschlossene äußere Kammerendwand (26), die in Querrichtung angeordnet ist und geeignet ist, senkrecht zur Öffnung (16) angeordnet zu werden, und mindestens eine Kammerumfangswand (27) aufweist, die sich ausgehend von ihrem distalen Endteil (27a) ausgehend vom Umfang (26a) der äußeren Endwand (26) in axialer Richtung in Richtung der proximalen Seite erstreckt,
- Mittel (14) zur Messung des Parameters, die starr an den Stütz- und Befestigungsmitteln (13) befestigt sind, die eine Messfläche (14a), die in der Kammer (17) angeordnet ist, die geeignet ist, elektrisch mit dem Inhalt (C) in Kontakt zu stehen, und elektrische Verbindungsmittel (15) aufweisen, die elektrisch mit den Messmitteln (14) verbunden sind, starr an den Stütz- und Befestigungsmitteln (13) befestigt sind, geeignet sind, von der Außenseite des Behälters (1) zugänglich zu sein, wenn die Messvorrichtung (12) daran befestigt ist, und elektrisch mit elektrischen Anschlussmitteln (20) verbunden zu sein, die sich auf der Außenseite des Behälters (1) befinden, **dadurch gekennzeichnet, dass** die Menge (23) von Wänden, die ein starres Ganzes bilden, eine vollständige, das heißt geschlossene, innere Kammerendwand (30) aufweist, die, ausgehend von dem proximalen axialen Endteil (27b) der Umfangswand (27), mindestens teilweise allgemein in Querrichtung senkrecht zur äußeren Endwand (26) angeordnet ist, wobei der offene Teil (18) in der Umfangswand (27) eingerichtet ist, wobei die Kammer (17) eine Ruhekammer (17) ist, die geeignet ist, einen Einschluss des Inhalts (C), der sich darin befindet, zu gewährleisten, die von dem Innenraum (3a) isoliert ist und durch die vollständige, das heißt geschlossene, innere Kammerendwand (30) von der darin herrschenden Bewegung geschützt ist, derart, dass die Einwirkung dieser Bewegung auf die Messung des Parameters des Inhalts (C) durch die Messfläche (14a) minimiert wird.

2. Messvorrichtung (12) nach Anspruch 1, wobei die Stütz- und Befestigungswand (22) mindestens teilweise eine allgemein ebene Form aufweist, die durch einen Umfangsaußenrand (24) abgegrenzt ist, von dem eine der Flächen (25a, 25b) eine Umfangszone (25c) zur starren und dichten Befestigung an der Wand (2) des Behälters (1) an dem Umfang (16a) seiner Öffnung (16) aufweist.

3. Messvorrichtung (12) nach einem der Ansprüche 1 und 2, wobei die Stütz- und Befestigungswand (22) und die äußere Endwand (26) zwei funktional unterschiedliche Teile einer selben Wand sind.

4. Messvorrichtung (12) nach einem der Ansprüche 1 bis 3, wobei die äußere Endwand (26) und die Stütz- und Befestigungswand (22) in allgemein axialer Richtung versetzt sind und durch eine Verbindungsumfangswand (29) miteinander verbunden sind, die sich mindestens teilweise allgemein in axialer Richtung erstreckt, sich in Bezug zur Stütz- und Befestigungswand (22) in Richtung der distalen Seite oder in Richtung der proximalen Seite erstreckt.

5. Messvorrichtung (12) nach einem der Ansprüche 1 bis 4, wobei die Messfläche (14a) sich vollständig in der Ruhekammer (17) auf mindestens einer Fläche der äußeren Endwand (26) und/oder einer Wand von der Menge (23) von Wänden befindet und der Innenseite der Ruhekammer (17) zugewandt ist.

6. Messvorrichtung (12) nach Anspruch 5, wobei die Messfläche (14a) sich auf der Umfangswand (27) und/oder auf der inneren Endwand (30) befindet.

7. Messvorrichtung (12) nach einem der Ansprüche 5 und 6, wobei die Messfläche (14a) sich senkrecht zu einem geschlossenen Teil der Menge (23) von Wänden und/oder der äußeren Endwand (26) befindet.

8. Messvorrichtung (12) nach einem der Ansprüche 5 bis 7, wobei die Messfläche (14a) im Wesentlichen eben und koplanar zur im Wesentlichen ebenen Innenfläche der Wand, auf der sie sich befindet, ist.

9. Messvorrichtung (12) nach einem der Ansprüche 5 bis 8, wobei die Messfläche (14a) sich in der Nähe eines geschlossenen Teils (19) einer gegenüberstehenden Wand von der Menge (23) von Wänden oder der äußeren Kammerendwand (26) befindet und/oder von einem offenen Teil (18) beabstandet ist.

10. Messvorrichtung (12) nach einem der Ansprüche 1 bis 9, die mehrere Mittel (14) zur Messung von einem oder mehreren Parametern aufweist.

11. Messvorrichtung (12) nach Anspruch 10, wobei die Messflächen (14a) von den mehreren Messflächen der mehreren Messmittel (14) sich auf der der Innenseite der Ruhekammer (17) zugewandten Fläche einer selben Wand (26, 27, 29, 30) oder unterschiedlicher Wände (26, 27, 29, 30) befinden.

12. Messvorrichtung (12) nach einem der Ansprüche 1 bis 11, wobei die elektrischen Verbindungsmittel (15) durch die Stütz- und Befestigungswand (22) und/oder die äußere Endwand (26) und/oder die Verbindungsumfangswand (29) getragen werden.

13. Messvorrichtung (12) nach einem der Ansprüche 1 bis 12, wobei die elektrischen Verbindungsmittel (15) in einem Schutzhohlraum (32) untergebracht sind.

14. Messvorrichtung (12) nach Anspruch 13, wobei der Schutzhohlraum (32) durch die äußere Endwand (26) und entweder die Verbindungsumfangswand (29) oder eine dazu bestimmte Wand (33) des Hohlraums abgegrenzt ist.

15. Messvorrichtung (12) nach einem der Ansprüche 1 bis 14, die mehrere elektrische Verbindungsmittel (15) aufweist.

16. Messvorrichtung (12) nach einem der Ansprüche 1 bis 15, wobei die Messmittel (14) Mittel (14) zur Messung der Leitfähigkeit, der Impedanz, der Kapazitanz, der Temperatur des biopharmazeutischen Inhalts (C) sind.

17. Messvorrichtung (12) nach einem der Ansprüche 1 bis 16, wobei die Stütz- und Befestigungswand (22) ein gewisses starres Verhalten aufweist.

18. Messvorrichtung (12) nach einem der Ansprüche 1 bis 17, **dadurch gekennzeichnet, dass** die Stütz- und Befestigungsmittel (13) mindestens ein Stütz- oder Führungsmittel (10) aufweisen, das geeignet ist, mindestens einen Drehzapfen (7) eines Mischmittels (6) zu stützen oder zu führen.

19. Messvorrichtung (12) nach Anspruch 18, **dadurch gekennzeichnet, dass** das mindestens eine Stütz- oder Führungsmittel (10) sich auf der Stütz- und Befestigungswand (22) und/oder der Menge (23) von Kammerwänden befindet oder dadurch getragen wird.

20. Messvorrichtung (12) nach Anspruch 19, **dadurch gekennzeichnet, dass** das mindestens eine Stütz- oder Führungsmittel (10) mit der Stütz- und Befestigungswand (22) und/oder der Menge (23) von Kammerwänden ein Endblindlager von mindestens einem Drehzapfen (7) eines Mischmittels (6) bildet.

21. Messvorrichtung (12) nach einem der Ansprüche 1 bis 20, **dadurch gekennzeichnet, dass** sie zum Einmalgebrauch ist.

22. Biopharmazeutischer Behälter (1) vom Typ, der einen oder mehrere Eingänge und Ausgänge (4), mindestens ein Mischmittel (6), gegebenenfalls mindestens ein Lüftungsmittel und einen Innenraum (3) aufweist, der durch eine Wand (2) abgegrenzt ist, die mindestens eine Durchgangs- und Montageöffnung (16) für mindestens eine Messvorrichtung (12) aufweist, wobei der biopharmazeutische Inhalt (C) des Behälters (1) einer Bewegung unterzogen werden kann, wobei die mindestens eine Messvorrichtung (12) starr und auf dichte Weise an dem Behälter (1) um die mindestens eine Durchgangsöffnung (16) herum befestigt ist, und geeignet ist, um einerseits mit dem Inhalt (C) und andererseits mit elektrischen Anschlussmitteln (20) elektrisch verbunden zu sein, die sich außerhalb des Behälters (1) befinden, wobei die mindestens eine Messvorrichtung (12) eine Messvorrichtung (12) nach einem der Ansprüche 1 bis 21 ist.

23. Biopharmazeutischer Behälter (1) nach Anspruch 22, wobei mindestens ein Mischmittel (6), gegebenenfalls mindestens ein Lüftungsmittel, mindestens ein Organ (9) aufweist, das sich im Innenraum (3) befindet.

24. Biopharmazeutischer Behälter (1) nach einem der Ansprüche 22 und 23, wobei eine Messvorrichtung (12) wenig von dem mindestens einen Mischmittel (6) und gegebenenfalls von dem mindestens einen Lüftungsmittel (11) entfernt ist.

25. Biopharmazeutischer Behälter (1) nach einem der Ansprüche 22 bis 24, wobei eine Messvorrichtung (12) sich im unteren Teil des biopharmazeutischen Behälters (1) befindet.

26. Biopharmazeutischer Behälter (1) nach einem der Ansprüche 22 bis 25, wobei die Wand (2) des biopharmazeutischen Behälters (1) eine gewisse Biegsamkeit aufweist.

27. Biopharmazeutischer Behälter (1) nach einem der Ansprüche 22 bis 26, **dadurch gekennzeichnet, dass** er zum Einmalgebrauch ist.

28. Biopharmazeutischer Behälter (1) nach einem der Ansprüche 22 bis 27, der ein Beutel zur Lagerung und/oder zum Auffüllen und/oder zur Handhabung und/oder zum Transport und/oder zum Mischen eines biopharmazeutischen Inhalts (C) oder ein Bioreaktor eines biopharmazeutischen Inhalts (C) ist.

## Claims

1. A device (12) for sensing a parameter related to an electrical phenomenon and able to be translated into an electrical signal, such as in a biopharmaceutical content (C) located in the space (3) of a biopharmaceutical container (1) delimited by a wall (2) having an opening (16), the content (C) being able to be subject to agitation, the sensing device (12) having an axis (YY), a proximal side towards the container (1) and a distal side opposite to the container (1), comprising:
support and fixing means (13) of a wall type, rigid, able to support the members constituting the sensing device (12) and to be fixed rigidly and sealingly to the wall (2) at the periphery (16a) of the opening (16), comprising a support and fixing wall (22) and a set (23) of chamber walls, delimiting a chamber (17) for the content (C), having a closed part (19) that includes an open part (18) able to provide passage of the content (C) between the chamber (17) and the internal space (3a) of the container (1) external to the chamber (17), said set (23) of walls comprising an external chamber end wall (26), closed, disposed transversely, able to come in line with the opening (16) and at least one peripheral chamber wall (27), extending from its distal end part (27a) in an axial direction towards the proximal side from the periphery (26a) of the external end wall (26), and
means (14) of capturing the parameter, rigidly fixed to the support and fixing means (13), comprising a capture face (14a) located in the chamber (17), able to be in electrical contact with the content (C), and electrical connection means (15) electrically connected to the sensing means (14), rigidly fixed to the support and fixing means (13), able to be accessible from the outside of the container (1) when the sensing device (12) is fixed to it and to be electrically connected to electrical connection means (20) located outside the container (1),
the set (23) of walls comprising an internal chamber end wall (30) forming a rigid whole, solid, that is to say closed, disposed at least in part generally transversely in line with the external end wall (26) from the proximal axial end part (27b) of the peripheral wall (27), the open part (18) being provided in the peripheral wall (27),
the chamber (17) being a holding chamber (17) able to provide confinement of the content (C) located therein, the chamber being isolated from the internal space (3a) and preserved from the agitation prevailing therein by the internal chamber end wall (30), solid, that is to say closed, so as to minimise the impact of said agitation on the measurement of the parameter of the content (C) by the capture face (14a).

2. A sensing device (12) according to claim 1, in which the support and fixing wall (22) is at least partly roughly flat in shape, delimited by a peripheral external edge (24), one of the faces (25a, 25b) of which comprises a peripheral area (25c) for rigid sealed fixing to the wall (2) of the container (1) at the periphery (16a) of its opening (16).

3. A sensing device (12) according to claim 1 or 2, in which the support and fixing wall (22) and the external end wall (26) are two functionally distinct parts of the same wall.

4. A sensing device (12) according to any of claims 1 to 3, in which the external end wall (26) and the support and fixing wall (22) are offset in a roughly axial direction and connected to each other by a peripheral connecting wall (29), extending at least partly roughly in the axial direction, extending with respect to the support and fixing wall (22) towards the distal side or towards the proximal side.

5. A sensing device (12) according to any of claims 1 to 4, in which the capture face (14a) is entirely located in the holding chamber (17) on at least one face of the external end wall (26) and/or of a wall in the set (23) of walls, and is turned towards the inside of the holding chamber (17).

6. A sensing device (12) according to claim 5, in which the capture face (14a) is located on the peripheral wall (27) and/or on the internal end wall (30).

7. A sensing device (12) according to claim 5 or 6, in which the capture face (14a) is located in line with a closed part of the set (23) of walls and/or of the external end wall (26).

8. A sensing device (12) according to any of claims 5 to 7, in which the capture face (14a) is substantially flat and coplanar with the substantially flat internal face of the wall on which it is located.

9. A sensing device (12) according to any of claims 5 to 8, in which the capture face (14a) is close to a closed part (19) of a wall facing the set (23) of walls or the external end wall (26) of the chamber and/or is distant from an open part (18).

10. A sensing device (12) according to any of claims 1 to 9, which comprises a plurality of means (14) of capturing one or more parameters.

11. A sensing device (12) according to claim 10, in which the captures faces (14a) in the plurality of capture faces of the plurality of capture means (14) are located on the face turned towards the inside of the holding chamber (17) of the same wall (26, 27, 29, 30) or different walls (26, 27, 29, 30).

12. A sensing device (12) according to any of claims 1 to 11, in which the electrical connection means (15) are carried by the support and fixing wall (22) and/or the external end wall (26) and/or the peripheral connecting wall (29).

13. A sensing device (12) according to any of claims 1 to 12, in which the electrical connection means (15) are housed in a protective cavity (32).

14. A sensing device (12) according to claim 13, in which the protective cavity (32) is delimited by the external end wall (26) and either the peripheral connecting wall (29) or a dedicated cavity wall (33).

15. A sensing device (12) according to any of claims 1 to 14, which comprises a plurality of electrical connection means (15).

16. A sensing device (12) according to any of claims 1 to 15, in which the capture means (14) are means (14) of sensing the conductivity, impedance, capacitance and temperature of the biopharmaceutical content (C).

17. A sensing device (12) according to any of claims 1 to 16, in which the support and fixing wall (22) has a certain rigid strength.

18. A sensing device (12) according to any of claims 1 to 17, in which the support and fixing means (13) include at least one support or guide means (10) able to support or guide at least one trunnion (7) of a mixing means (6).

19. A sensing device (12) according to claim 18, in which the at least one support or guide means (10) is located on or carried by the support and fixing wall (22) and/or the set (23) of chamber walls.

20. A sensing device (12) according to claim 19, in which the at least one support or guide means (10) forms, with the support and fixing wall (22) and/or the set (23) of chamber walls, a blind end bearing for at least one trunnion (7) of a mixing means (6).

21. A sensing device (12) according to any of claims 1 to 20, the sensing device being for single use.

22. A biopharmaceutical container (1) of the type comprising one or more inlets and outlets (4), at least one mixing means (6), when applicable at least one aeration means, an internal space (3) delimited by a wall (2) having at least one passage and mounting opening (16) for at least one sensing device (12), the biopharmaceutical content (C) of the container (1) being able to be subject to agitation, the at least one sensing device (12) being fixed rigidly and sealingly to the container (1) around the at least one passage opening (16) and able to be in electrical communication firstly with the content (C) and secondly with electrical connection means (20) located outside the container (1), in which the at least one sensing device (12) is a sensing device according to any of claims 1 to 21.

23. A biopharmaceutical container (1) according to claim 22, in which the at least one mixing means (6) and where applicable the at least one aeration means comprise at least one member (9) located in the internal space (3).

24. A biopharmaceutical container (1) according to claim 22 or 23, in which a sensing device (12) is close to the at least one mixing means (6) and where applicable to the at least one aeration means (11).

25. A biopharmaceutical container (1) according to any of claims 22 to 24, in which a sensing device (12) is located at the bottom part of the biopharmaceutical container (1).

26. A biopharmaceutical container (1) according to any of claims 22 to 25, in which the wall (2) of the biopharmaceutical container (1) has a certain flexibility.

27. A biopharmaceutical container (1) according to any of claims 22 to 26, the biopharmaceutical container being for single use.

28. A biopharmaceutical container (1) according to any of claims 22 to 27, which constitutes a pouch for storing and/or filling and/or manipulating and/or transporting and/or mixing a biopharmaceutical content (C) or a bioreactor for a biopharmaceutical content (C).
